# EUROPEAN PATENT APPLICATION

(11) **EP 1 306 430 A1**
(43) Date of publication of application: **02.05.2003**
(21) Application number: 01984391.1
(22) Date of filing: 30.07.2001
(51) Int. Cl.: C12N 15/09, C12P 21/02

(54) **PROCESS FOR PRODUCING RECOMBINED PROTEIN**

(30) Priority: 31.07.2000 JP 2000232389
(71) Applicant: Takeda Chemical Industries, Ltd., Osaka-shi, Osaka 541-0045 (JP)
(72) Inventor: ITO, Takashi,, Kobe-shi,Hyogo 658-0053 (JP); KOBAYASHI, Masayuki, Kawabe-gun, Hyogo 666-0261 (JP); SAWADA, Hidekazu, Neyagawa-shi, Osaka 572-0806 (JP)
(74) Representative: Rickard, Timothy Mark Adrian
(86) International application number: JP0106531
(87) International publication number: WO02010370

(57) **Abstract**

It is intended to provide a process for efficiently producing a recombinant protein free from the N-terminal methionine. In culturing a host transformed by an expression vector for expressing a gene-modified protein, the dissolved oxygen concentration in the liquid culture medium is controlled to about 1 ppm or lower. Thus, the ratio of the expression of a recombinant protein free from the N-terminal methionine is elevated compared with the case wherein no such control is performed.

## Description

### TECHNICAL FIELD

The present invention relates to a process for producing recombinant proteins.

### BACKGROUND ART

*Escherichia coli* is used most widely as a host for producing gene recombinant proteins (hereinafter, sometimes referred to just "recombinant protein(s)") from the viewpoint of cost and so forth. However, when a recombinant protein is expressed in *E. coli*, accurate processing as seen when the protein is expressed in an animal cell may not occur. The addition of methionine (derived from the initiation codon ATG) to the N-terminal, which phenomenon is seen when a recombinant protein is expressed in *E. coli*, is one example of such inaccurate processing.

Removal of the methionine added to the N-terminal of a recombinant protein expressed in *E. coli* is usually performed with methionine aminopeptidase. However, this enzyme hardly works when the amino acid residue following the methionine is arginine, proline or the like. Although this enzyme works relatively well when the amino acid residue following the methionine is serine, alanine or the like, most part of the expressed recombinant protein retains the N-terminal methionine in many cases where the expression level of the protein is high. Although it may be sometimes possible to separate a recombinant protein with N-terminal methionine from the recombinant protein without N-terminal methionine by purification, usually it is difficult to separate these two proteins that are different only in the presence or absence of N-terminal methionine. This is disadvantageous in terms of purification yield. Thus, decreasing the ratio of a recombinant protein with N-terminal methionine is also expected to be effective in improving purification yield.

Recombinant proteins in which methionine is added to the N-terminal may not exhibit their intrinsic activities, or may have problems of antigenicity when they are used as medicines. As to other methods for removing the N-terminal methionine or producing a recombinant protein without addition of such methionine include a method in which secretion expression is carried out (C.N. Chang et al., Gene 55:189-196 (1987)); a method in which a recombinant protein is expressed as a fusion gene (Itakura et al., Science 198:1056 (1977)); a method in which methionine is removed with amino peptidase after the expression of a recombinant protein (Nakagawa et al., Biotechnology 5:824 (1987)) and so forth are known. However, the method of secretion expression has a drawback that the expression level is lower than usual expression. With respect to the method of expression as a fusion protein and the method of removing methionine with aminopeptidase after expression, an operation to cleave the fusion protein or to remove the methionine is required after expression of the recombinant protein. This not only makes the production procedures complicated but also reduces the final yield and/or purity; thus, these methods are disadvantageous.

Establishment of a cultivation process for *E. coli* as a host for efficiently expressing a recombinant protein from which the N-terminal methionine has been removed even under high expression has been a problem to be solved.

### DISCLOSURE OF THE INVENTION

The present inventors have found that a recombinant protein from which the N-terminal methionine has been removed can be expressed highly efficiently by controlling the dissolved oxygen concentration in the culture broth at about 1 ppm or below and retaining the concentration at such a low level in the cultivation of *E. coli* transformed with an expression vector for expressing the gene recombinant protein. Thus, the present invention has been achieved.

The present invention provides the following process:
(1) A process of producing a recombinant protein in which methionine is not added to the N-terminal comprising culturing a host transformed with an expression vector for expressing the recombinant protein, wherein the process is characterized by controlling the dissolved oxygen concentration in the culture broth at about 1 ppm or below.
(2) The process of (1) above, wherein the dissolved oxygen concentration in the culture broth is about 0.01-1.0 ppm.
(3) The process of (1) above, wherein the dissolved oxygen concentration in the culture broth is 0.05-1.0 ppm.
(4) The process of (1) above, wherein the dissolved oxygen concentration in the culture broth is controlled at about I ppm or below by regulating the agitation speed of the culture broth.
(5) The process of (I) above, wherein the host is a bacterium belonging to the genus *Escherichia.*
(6) The process of (1) above, wherein an expression inducer is added to the culture broth.
(7) The process of (6) above, wherein the expression inducer is isopropyl- β-D-thio-galactopyranoside.
(8) The process of (6) above, wherein the concentration of the expression inducer in the culture broth is about 0.01-1 mM.

### BRIEF DESCRIPTION OF THE DRAWINGS

Fig. 1 is a construction diagram for plasmid pTCPTH2.
Fig. 2 shows the reversed-phase chromatogram obtained in Example 1. "Des-Met form" indicates the peak of [Cys³⁵]-human PTH (1-84), and "Met addition form" indicates the peak of the Met-addition form of [Cys³⁵]-human PTH (1-84).
Fig. 3 shows the yields of [Cys³⁵]-human PTH (1-84) and the Met-addition form thereof at 24 hrs after the start of the cultivation carried out in Example 1. "□" represents the yield of [Cys³⁵]-human PTH (1-84), and "■" represents the yield of the Met-addition form of [Cys³⁵]-human PTH (1-84).
Fig. 4 shows the time course of the cultivation carried out in Example 2. In the upper graph, "―" represents the dissolved oxygen concentration and "···" represents the agitation speed. In the lower graph, "○" represents the yield of [Cys³⁵]-human PTH (1-84); "●" represents the Met-addition form of [Cys³⁵]-human PTH (1-84); and "□" represents the turbidity of the culture broth (in Klett unit (KU)).
Fig. 5 shows a construction diagram for plasmid pGFPNP.
Fig. 6 shows a construction diagram for plasmid pGFPNPRG.
Fig. 7 shows a construction diagram for plasmid pTFCRGAL.
Fig. 8 shows a construction diagram for plasmid pTFCS4.

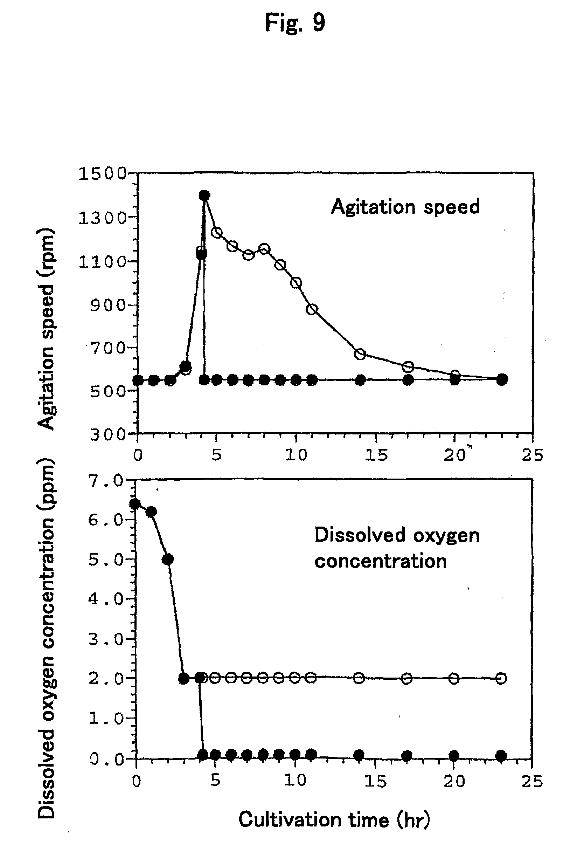

Fig. 9 shows the time course of the cultivation carried out in Example 4. In the upper graph, "○" represents the agitation speed when the cultivation was continued while controlling the dissolved oxygen concentration at 2.0 ppm, and "●" represents the agitation speed when the cultivation was continued while fixing the agitation speed at 550 rpm. In the lower graph, "○" represents the dissolved oxygen concentration when the cultivation was continued while controlling the dissolved oxygen concentration at 2.0 ppm, and "●" represents the dissolved oxygen concentration when the cultivation was continued while fixing the agitation speed at 550 rpm.
Fig. 10 shows the results of electrophoresis of the culture broth taken at indicated time points carried out in Example 4. The expression "Controlled at 2.0 ppm" shows the culture broth when the cultivation was continued while controlling the dissolved oxygen concentration at 2.0 ppm. The expression "Agitation at 550 rpm" shows the culture broth when the cultivation was continued while fixing the agitation speed at 550 rpm. The figures 7, 10, 14, 17 and 20 represent hours after the start of the cultivation.
Fig. 11 shows ratios of rat GALP-CS23 fusion protein and the Met-addition form thereof at 17 and 24 hrs after the start of the cultivation carried out in Example 4. "□" represents the production ratio (%) of rat GALP-CS23 fusion protein, and "■" represents the production ratio of the Met-addition form of rat GALP-CS23 fusion protein.

### BEST MODES FOR CARRYING OUT THE INVENTION

According to the present invention, it is possible to obtain a protein of interest in which methionine is not added to the N-terminal efficiently by introducing a gene of the protein of interest into a prokaryotic host (such as *E. coli*) and culturing the recombinant prokaryote (preferably *E. coli*) while controlling the dissolved oxygen concentration in the culture broth at about 1 ppm or below.

In the present specification, a protein of interest is written with the N-terminal (amino terminal) on the left end and the C-terminal (carboxyl terminal) on the right end according to the convention in peptide representation.

Specific examples of proteins of interest include eukaryote-derived proteins such as growth factors (e.g. FGF-9, etc.), interleukins (e.g. interleukin-2, interleukin-6, etc.), interferons, chemokines (e.g. RANTES, MIP-1 α, MCP-1, etc.), physiologically active peptides [e.g. parathyroid hormone (PTH), ligand peptide for galanin receptor (GALP) (WO 99/48920), KiSS-1 peptide (WO 00/24890), RFRP-1 (WO 00/29441), PrRP (WO 97/24436), angiotensin, bradykinin, calcitonin, conantokin, corticotropin-releasing factor, dynorphin, endorphin, enkephalin, galanin, etc.], neurotrophic factors (e.g. GDNF, etc.) and enzymes (e.g. LCAT-like lysophospholipase, etc.). These proteins may be used by themselves or in the form of a fusion protein where other eukaryote-derived protein [e.g. basic fibroblast growth factor (bFGF), bFGF derivative, glutathione-S-transferase (GST), maltose-binding protein (MBP), Green Fluorescent Protein (GFP), etc.], a prokaryote-derived protein or a tag such as polyhistidine is fused to the C-terminal.

The protein of interest may be in the form of a salt. As salts of a protein of interest, physiologically acceptable salts are particularly preferable. Examples of such salts useful in the invention include acid-addition salts such as salts formed with inorganic acids (e.g. hydrochloric acid, phosphoric acid, hydrobromic acid or sulfuric acid) and salts formed with organic acids (e.g. acetic acid, formic acid, propionic acid, fumaric acid, maleic acid, succinic acid, tartaric acid, citric acid, malic acid, oxalic acid, benzoic acid, methanesulfonic acid or benzenesulfonic acid).

As a vector for introducing a gene of a protein of interest into a host, any vector may be used as long as it is replicable in prokaryotes such as bacteria belonging to the genus *Escherichia*. Specific examples of useful vectors include pBR322 [Gene 2:95 (1977)], pBR313 [Gene 2:75 (1977)], pBR324, pBR325 [Gene 4:121 (1978)], pBR327, pBR328 [Gene 9:287 (1980)], pBR329 [Gene 17:79 (1982)], pKY2289 [Gene 3:1 (1978)], pKY2700 [Biochemistry 52:770 (1980)], pACYC177, pACYC184 [Journal of Bacteriology 134:1141 (1978)], pRK248, pRK646, pDF [Methods in Enzymology 68:268 (1979)], pUC12 [Gene 19:259 (1982)], pUC13 [Gene 19:259 (1982)], pUC18 and pUC19 [Yanisch-Perron et al., Gene 33:103 (1985)].

As a promoter for transcription of the gene of a protein of interest, trp promoter, lac promoter, recA promoter, λP_{L} promoter, lpp promoter, T7 promoter, tac promoter or the like may be enumerated. If necessary, SD (Shine Dalganro) sequence may be inserted down stream of the promoter.

When a T7 promoter system is used, any of the 17 promoters found on T7 DNA [J.L. Oakley et al., Proc. Natl. Acad. Sci. USA , 74:4266-4270 (1977); M.D. Rosa, Cell 16:815-825 (1979); N. Panayotatos et al., Nature 280:35 (1979); J.J. Dunn et al., J. Mol. Biol. 166:477-535 (1983)] may be used as T7 promoter. Preferably, φ 10 promoter [A.H. Rosenberg et al., Gene 56:125-135 (1987)] is used.

A promoter DNA may be inserted into an appropriate site of a vector according to known methods. For insertion of the DNA encoding a protein of interest, appropriate restriction enzyme recognition sites may be provided usually downstream of the promoter DNA.

As a transcription terminator, a terminator functional in *E. coli* systems may be used. Preferably, T φ terminator [F.W. Studier et al., J. Mol. Biol. 189:113-130 (1986)] is used.

As a T7 RNA polymerase gene, T7 gene [F.W. Studier et al., J. Mol. Biol. 189:113-130 (1986)] may be used.

The gene of a protein of interest may be inserted into a vector downstream of the promoter DNA according to known methods.

The vector may, if desired, further comprise selective markers. Examples of useful selective markers include ampicillin resistance gene (Amp^{r}), tetracycline resistance gene and kanamycin resistance gene, etc.

A transformant can be prepared by transforming a host with a recombinant vector integrating the DNA encoding a protein of interest.

As the host, prokaryotes such as bacteria belonging to the genus *Escherichia* may be used.

Specific examples of bacteria belonging to the genus *Escherichia* useful in the invention include *E. coli* K12, DH1 [Proc. Natl. Acad. Sci. USA, Vol. 60, 160 (1968)], JM103 [Nucleic Acids Research, Vol. 9, 309 (1981)], JA221 [Journal of Molecular Biology, Vol. 120, 517 (1978)], HB101 [Journal of Molecular Biology, Vol. 41, 459 (1969)], C600 [Genetics, Vol. 39, 440 (1954)], MM294, JM109 and BL21. For example, when a T7 promoter system is used, any of the above-mentioned *E. coli* strains in which T7 RNA polymerase DNA (T7 DNA1) [F.W. Studier et al., J. Mol. Biol. 189:113-130 (1986)] is integrated alone or together with other plasmid may be used. Preferably, MM294 (DE3), JM109 (DE3), BL21 (DE3) or the like in which T7 DNA1-integrating λ phage is lysogenized may be used.

The transformation of bacteria belonging to the genus *Escherichia* may be carried out according to methods described, for example, in Proc. Natl. Acad. Sci. USA, Vol. 69, 2110 (1972) or Gene Vol. 17, 107 (1982).

When a transformant of a prokaryote is cultured, a liquid medium is appropriate as a medium for culturing transformants for the purpose of producing a protein of interest. The medium is allowed to contain carbon sources, nitrogen sources, minerals, and so on that are necessary for the growth of the transformant. As carbon sources, glucose, dextrin, soluble starch, sucrose or the like may be enumerated. As nitrogen sources, organic or inorganic substances such as ammonium salts, nitrates, corn steep liquor, peptone, casein, meat extract, bean cake, potato extract or the like may be enumerated. As minerals, calcium chloride, sodium dihydrogenphosphate, magnesium chloride or the like may be enumerated. Further, yeast extract, vitamins, growth-promoting factors, etc. may also be added to the medium. The pH of the medium is preferably about 5-8, more preferably about 6-8.

As a medium to culture bacteria of the genus *Escherichia*, M9 medium containing glucose and casamino acids [Miller, Journal of Experiments in Molecular Genetics, 431-433, Cold Spring Harbor Laboratory, New York, (1972)] is preferable, for example.

If necessary, an expression inducer may be added to the medium in order to allow the promoter to work efficiently.

Examples of expression inducers include isopropyl- β-D-thiogalactopyranoside (TPTG) and 3 β-indolyl acrylic acid. These substances may be used at a concentration of about 0.01-1.5 mM, preferably about 0.01-1 mM. The time of addition of such an expression inducer may be either before or after the controlling of the dissolved oxygen concentration in the culture broth at about 1 ppm or below. Preferably, the inducer is added before the controlling.

IPTG is used when, for example, lac promoter or tac promoter is used. Even when T7 RNA polymerase DNA (T7 DNA1) is ligated downstream of lac promoter in a T7 promoter system, IPTG is used as an expression inducer.

3β-Indolyl acrylic acid is used when, for example, trp promoter is used. In order to allow recA promoter to work more efficiently (i.e., to decrease the inhibitory function against the expression of recA gene), if necessary, drugs such a mitomycin C or nalidixic acid may be added, or ultraviolet irradiation may be applied, or the pH of the culture broth may be changed to the alkaline side.

Induction of expression may be performed not only by adding an expression inducer but also by changing the cultivation temperature. When a recombinant carrying an expression vector comprising λ cIts repressor and λPL promoter is used, it is preferable to culture the recombinant at about 15-36°C, preferably at 30-36°C, for example, and to induce the expression of a protein of interest by inactivation of the λ cIts repressor at about 37-42°C, for example. In a T7 promoter system also, if T7 RNA polymerase DNA (T7 DNA1) is ligated downstream of λPL promoter, T7 promoter is specifically activated by raising the cultivation temperature to thereby generate T7 phage RNA polymerase 1.

The cultivation of a transformant is carried out usually at about 15-43°C, preferably at about 25-43°C for about 3-24 hrs. If necessary, aeration or agitation may be employed.

As a method for controlling the dissolved oxygen concentration in the culture broth at about 1 ppm or below, a method may be given in which agitation speed, aeration rate, the type of feed gas, etc. are selected. These conditions may be regulated independently or in combination. Among all, control of the dissolved oxygen concentration by regulating the agitation speed is preferable. Since these conditions vary depending on the cell density at the time of induction of expression, the concentration of the expression inducer, the size of the fermenter, the shape of the agitation impeller, etc., they may be selected appropriately so that the dissolved oxygen concentration is retained at 1 ppm. Further, the dissolved oxygen concentration may be monitored and the agitation speed, aeration rate or the oxygen partial pressure of the feed gas may be automatically controlled so that the dissolved oxygen concentration is maintained at a preferable level. These controls may be performed continuously or in a step-wise manner.

Specifically, when a 3 to 5 L fermenter is used, for example, the dissolved oxygen concentration can be controlled to come to the intended level by culturing at about 37°C with aeration of 1-3 L/min and by agitating at a speed of about 300-700 rpm. By increasing the agitation speed or aeration rate, it is possible to increase the dissolved oxygen concentration. Contrarily, by decreasing the agitation speed or aeration rate, it is possible to decrease the dissolved oxygen concentration.

The dissolved oxygen concentration maintained during cultivation in the present invention may be any level as long as it gives an increased ratio of a recombinant protein without N-terminal Met addition. The dissolved oxygen concentration may be changed to any level as long as a recombinant protein without N-terminal Met addition is obtained more than the recombinant protein with N-terminal Met addition (e.g. about 60% or more, preferably about 70% or more, more preferably about 80% or more of the total protein obtained is the recombinant protein without N-terminal Met addition) at that level. Also, as long as the transformed cell retains growth suitable for the production of the protein of interest, it is preferable to maintain the dissolved oxygen concentration at a low level. For example, the dissolved oxygen concentration is maintained at about 1 ppm or below, preferably at 0.7 ppm or below, more preferable at 0.5 ppm or below, and is maintained, for example, at about 0.01 ppm or above, preferably at about 0.05 ppm or above, more preferably at about 0.1 ppm or above. The preferable range of the dissolved oxygen concentration in the culture broth is, for example, about 0.01-1.0 ppm, preferably about 0.05-1.0 ppm, more preferably about 0.05-0.7 ppm, still more preferably about 0.1-0.5 ppm.

Likewise, prokaryotes other than bacteria belonging to the genus *Escherichia* may be transformed and cultured.

Thus, by controlling the dissolved oxygen concentration, it is possible to produce a recombinant protein without N-terminal Met addition usually at a ratio of about 60% or more, preferably about 70% or more, more preferably about 80% or more in the total recombinant protein obtained.

Separation and purification of the protein of interest from the resultant culture can be carried out, for example, according to the methods described below.

For extraction of the protein of interest from cultured cells, the cells are harvested by known methods after the cultivation, suspended in a suitable buffer, and disrupted by sonication or by lysozyme and/or freezing and thawing, etc. Then, a crude extract of the protein is obtained by centrifugation or filtration. The buffer may contain a protein denaturing agent such as urea or guanidine hydrochloride, or a surfactant such as Triton X-100™. If the protein of interest is secreted into the culture broth, the supernatant is separated from the microorganisms or cells after completion of the cultivation and collected by known methods.

Purification of the protein of interest contained in the thus obtained culture supernatant or extract can be performed by an appropriate combination of known methods for separation and purification. These known methods include methods utilizing solubility (such as salting out or sedimentation with solvents), methods mainly utilizing difference in molecular weight (such as dialysis, ultrafiltration, gel filtration and SDS-polyacrylamide gel electrophoresis), methods utilizing difference in electric charge (such as ion-exchange chromatography), methods utilizing specific affinity (such as affinity chromatography), methods utilizing difference in the hydrophobicity (such as reversed-phase high-performance liquid chromatography), and methods utilizing difference in isoelectric point (such as isoelectric electrophoresis).

The recombinant protein of interest in which methionine is not added to the N-terminal may be separated/purified from the recombinant protein in which methionine is added to the N-terminal using known methods. However, the protein of interest may also be used as it is without separation/purification.

When the thus obtained protein of interest is a free protein, the protein can be converted into the above-described salt by known methods or methods based thereon. Contrarily, when the protein of interest is obtained in a salt form, the salt can be converted into a free protein or another salt according to known methods or methods based thereon.

The protein of interest produced by a recombinant host can be arbitrarily modified or a part thereof can be removed therefrom by using an appropriate protein modification enzyme for by chemical reaction before or after the purification. Examples of such enzymes include trypsin, chymotrypsin, arginyl endopeptidase and protein kinase. Examples of chemical reactions include cleavage reaction using bromocyan and cleavage reaction using 2-nitro-5-thiocyanobenzoic acid.

The extracted or separated/purified protein of interest is subjected to refolding, if necessary. Refolding can be performed by known methods described, for example, in Folding of Proteins, R.H. Pain (Ed.), 245-279 (1995) published by Springer-Verlag Tokyo or methods based thereon. Refolding can be performed, for example, by one-step or multi-step dilution in a buffer with no extraction agents (e.g. chaotropic solubilizers such as guanidine hydrochloride and urea, surfactants such as n-lauryl methyl glycine and SDS) or with extraction agents at low concentrations, dialysis with a semipermeable membrane, or replacement of a buffer using gel filtration. In order to prevent the aggregation of the protein of interest, arginine, polyethylene glycol, neutral surfactants, etc. may be added. For the formation of disulfide bonds in the protein, air oxidation, an oxidation-reduction buffer system, etc. may be employed. Examples of oxidation-reduction buffers include those based on glutathione, cysteine, dithiothreitol, 2-mercaptoethanol or cysteamine.

The resultant protein of interest may be used as a medicine, reagent, raw material, feed and so on depending on the nature of the protein.

When the protein of interest is used as a medicine, the protein may be used in a conventional manner. For example, the protein of interest may be used orally in the form of tablets (sugar-coated or enteric coated, if necessary), capsules, elixirs, microcapsules or the like; or parenterally in the form of injections such as aseptic solutions or suspensions in water or other pharmaceutically acceptable liquids. These preparations may be produced, for example, by mixing the compound or a salt thereof with physiologically acceptable carriers, flavoring agents, excipients, vehicles, antiseptics, stabilizers, binders, etc. in unit dosage forms. The amounts of active ingredients in these formulations are decided so that an appropriate dose within the specified range can be obtained.

Examples of additives which may be mixed in tablets, capsules, etc. include binders such as gelatin, corn starch, tragacanth gum and gum arabic, excipients such as crystalline cellulose, swelling agents such as corn starch, gelatin and alginic acid, lubricants such as magnesium stearate, sweetening agents such as sucrose, lactose and saccharin, and flavoring agents such as peppermint, akamono oil and cherry. When the unit dosage form is a capsule, liquid carriers such as oils and fats may further be included in addition to the above-mentioned materials. Sterile compositions for injection can be formulated according to conventional practices in pharmaceutical manufacturing, e.g., by dissolving or suspending active ingredients in vehicles such as water for injection, naturally occurring vegetable oils such as sesame oil, coconut oil, etc.

Examples of aqueous liquids for injection include physiological saline and isotonic solutions containing glucose and other auxiliary agents (e.g. D-sorbitol, D-mannitol, sodium chloride, etc.). They may be used in combination with a suitable auxiliary solubilizer such as alcohol (e.g. ethanol), polyalcohol (e.g. propylene glycol, polyethylene glycol), nonionic surface-active agent (e.g. Polysorbate 80™, HCO-50). Examples of oily liquids for injection include sesame oil, soybean oil, etc. They may be used in combination with an auxiliary solubilizer such as benzyl benzoate, benzyl alcohol, etc.

In addition, buffers (e.g. phosphate buffer, sodium acetate buffer, etc.), analgesic agents (e.g. benzalkonium chloride, procaine hydrochloride), stabilizers (e.g. human serum albumin, polyethylene glycol, etc.), preservatives (e.g. benzyl alcohol, phenol, etc.), antioxidants, etc. may also be admixed therewith. Usually, the prepared injections are filled in appropriate ampoules.

The thus obtained preparations may be administered to, for example, mammals (e.g. human, mouse, rat, guinea pig, rabbit, sheep, pig, bovine, cat, dog, monkey, etc.).

In the specification and drawings of the present application, the abbreviations used for bases (nucleotides), amino acids and so forth are those recommended by the IUPAC-TUB Commission on Biochemical Nomenclature or those conventionally used in the art. Examples of such abbreviations are given below. Amino acids which may have optical isomers are intended to represent their L-isomer unless otherwise specified.
DNA: Deoxyribonucleic acid
cDNA: Complementary deoxyribonucleic acid
A: Adenine
T: Thymine
G: Guanine
C: Cytosine
M: A or C
R: G or A
W: A or T
S: G or C
Y: T or C
K: G or T
V: A or G or C
H: A or C or T
D: A or G or T
B: G or C or T
N: A or C or G or T
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediaminetetracetic acid
SDS: Sodium dodecyl sulfate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine

The SEQ ID NOS of the SEQUENCE LISTING of the present specification show the sequences as indicated below.

### [SEQ ID NO: 1]

This shows the nucleotide sequence of primer 1 used in Reference Example 2.

### [SEQ ID NO: 2]

This shows the nucleotide sequence of primer 2 used in Reference Example 2.

### [SEQ ID NO: 3]

This shows the nucleotide sequence of synthetic DNA 1 used in Reference Example 2.

### [SEQ ID NO: 4]

This shows the nucleotide sequence of synthetic DNA 2 used in Reference Example 2.

### [SEQ ID NO: 5]

This shows the nucleotide sequence of synthetic DNA 3 used in Reference Example 2.

### [SEQ ID NO: 6]

This shows the nucleotide sequence of synthetic DNA 4 used in Reference Example 2.

### [SEQ ID NO: 7]

This shows the nucleotide sequence of synthetic DNA 5 used in Reference Example 3.

### [SEQ ID NO: 8]

This shows the nucleotide sequence of synthetic DNA 6 used in Reference Example 3.

### [SEQ ID NO: 9]

This shows the nucleotide sequence of primer 3 used in Reference Example 3,

### [SEQ ID NO: 10]

This shows the nucleotide sequence of primer 4 used in Reference Example 3.

### [SEQ ID NO: 11]

This shows the nucleotide sequence of primer 5 used in Reference Example 3.

### [SEQ ID NO: 12]

This shows the nucleotide sequence of primer 6 used in Reference Example 3.

### [SEQ ID NO: 13]

This shows the nucleotide sequence of primer 7 used in Reference Example 3.

### [SEQ ID NO: 14]

This shows the amino acid sequence of [Cys³⁵]-human PTH (1-84).

### [SEQ ID NO: 15]

This shows the nucleotide sequence of a gene encoding [Cys³⁵]-human PTH (1-84).

### [SEQ ID NO: 16]

This shows the amino acid sequence of human PTH (1-34).

### [SEQ ID NO: 17]

This shows the amino acid sequence of rat GALP-CS23 fusion protein.

### [SEQ ID NO: 18]

This shows the nucleotide sequence of a gene encoding rat GALP-CS23 fusion protein.

*Escherichia coli* MM294 (DE3)/pTCPTH, a transformant obtained in Reference Example 1 described later, has been deposited with the International Patent Organism Depository, National Institute of Advanced Industrial Science and Technology (former designation: National Institute of Bioscience and Human Technology, Agency of Industrial Science and Technology, Ministry of International Trade and Industry (NIBH)) located at Central 6, 1-1 Higashi 1-chome, Tsukuba City, Ibaraki Pref., Japan (Zip Code No. 305-8566) since July 27, 2000 under the Accession No. FERM BP-7245, and with the Institute for Fermentation, Osaka (IFO) located at 17-85 Jusanboncho 2-chome, Yodogawa-ku, Osaka City, Osaka Pref., Japan since January 23, 1998 under the Accession No. IFO 16156.

*Escherichia coli* JM109/pGFPNP, a transformant obtained in Reference Example 2 described later, has been deposited with the International Patent Organism Depository, National Institute of Advanced Industrial Science and Technology (former designation: NIBH) located at Central 6, 1-1 Higashi 1-chome, Tsukuba City, Ibaraki Pref., Japan (Zip Code No. 305-8566) since July 17, 2000 under the Accession No. FERM BP-7223, and with the Institute for Fermentation, Osaka (IFO) since April 24, 2000 under the Accession No. IFO 16426.

*Escherichia coli* MM294 (DE3)/pTFCRGAL, a transformant obtained in Reference Example 4 described later, has been deposited with the International Patent Organism Depository, National Institute of Advanced Industrial Science and Technology (former designation: NIBH) located at Central 6, 1-1 Higashi 1-chome, Tsukuba City, Ibaraki Pref., Japan (Zip Code No. 305-8566) since July 17, 2000 under the Accession No. FERM BP-7226, and with the Institute for Fermentation, Osaka (IFO) since April 24, 2000 under the Accession No. IFO 16429.

*Escherichia coli* MM294 (DE3)/pTFCS4, a transformant obtained in Reference Example 5 described later, has been deposited with the International Patent Organism Depository, National Institute of Advanced Industrial Science and Technology (former designation: NIBH) located at Central 6, 1-1 Higashi 1-chome, Tsukuba City, Ibaraki Pref., Japan (Zip Code No. 305-8566) since July 17, 2000 under the Accession No. FERM BP-7227, and with the Institute for Fermentation, Osaka (TFO) since April 24, 2000 under the Accession No. IFO 16430.

*Escherichia coli* (MM294 (DE3), pTCHGH-Na), which is a transformant carrying plasmid pTCHGH-Na that is used in Example 2 described later, has been deposited with the International Patent Organism Depository, National Institute of Advanced Industrial Science and Technology (former designation: NIBH) located at Central 6, 1-1 Higashi 1-chome, Tsukuba City, Ibaraki Pref., Japan (Zip Code No. 305-8566) since December 10, 1997 under the Accession No. FERM BP-6888, and with the Institute for Fermentation, Osaka (IFO) since October 16, 1997 under the Accession No. IFO 16124.

### EXAMPLES

### REFERENCE EXAMPLE 1

### Preparation of [Cys³⁵]-Human PTH (1-84)-Producing Recombinant E. coli

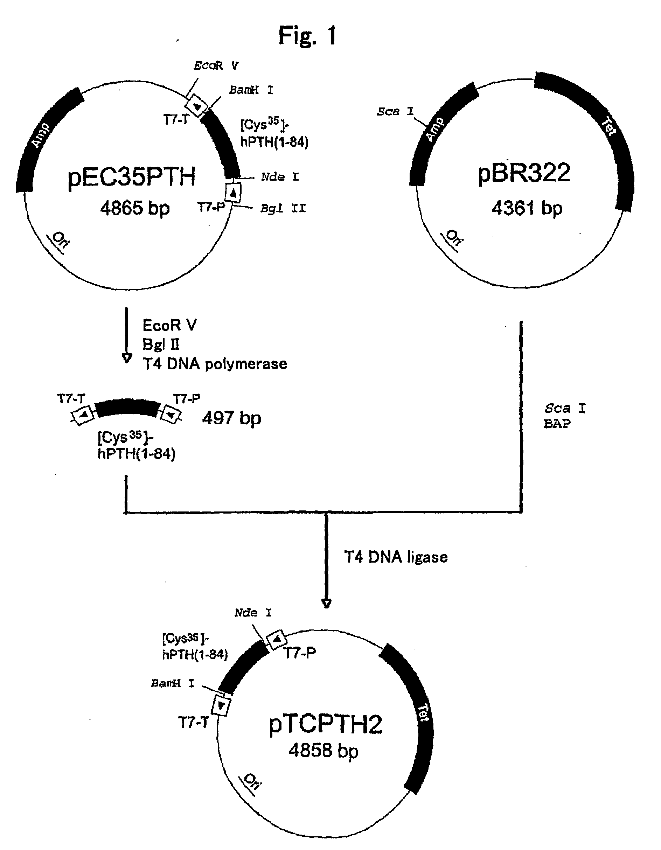

Plasmid pE-C35PTH was isolated from *E. coli* MM294 (DE3)/pE-C35PTH (Japanese Unexamined Patent Publication No. 5-304976) according to conventional methods. The isolated plasmid pE-C35PTH was completely digested with BglII and EcoRV to thereby obtain a DNA fragment comprising [Cys³⁵]-human PTH (1-84) gene, T7 promoter and T7 terminator. This DNA fragment was blunt-ended with T4 DNA polymerase. The resultant DNA fragment was ligated to Scal-digested pBR322 with T4 DNA ligase to thereby construct an expression plasmid pTCPTH2 having tetracycline resistance as a marker (Fig. 1).

Plasmid pTCPTH2 was introduced into *E. coli* MM294 (DE3) to thereby obtain *E. coli* MM294 (DE3)/pE-C35PTH.

### EXAMPLE 1

### Cultivation of [Cys³⁵]-Human PTH (1-84)-Producing Recombinant E. coli in 3 L-Jars

*E. coli* MM294 (DE3)/pTCPTH2 was cultured in 1 L of LB medium (1% peptone, 0.5% yeast extract, 0.5% sodium chloride) containing 10 mg/ml tetracycline at 30°C for 16 hrs under shaking. The resultant culture broth was transferred into 3-L jars each containing 1.5 L of a medium for primary fermentation (1.68% sodium monohydrogenphosphate, 0.3% sodium dihydrogenphosphate, 0.1% ammonium chloride, 0.05% sodium chloride, 0.024% magnesium sulfate, 0.02% New Pole LB-625, 0.0005% thiamine hydrochloride, 1.5% glucose, and 1.5% casamino acids). Then, aeration agitation culture was started at 37°C at an aeration rate of 1.5 L/min and at an agitation speed of 500 rpm. When the turbidity of the culture broth reached approx. 500 Klett units, isopropyl-β-D-thiogalactopyranoside (IPTG) was added at 5.95 mg/L min. From immediately after the addition of IPTG, cells were cultured while controlling the agitation speed in individual jars so that dissolved oxygen concentrations are retained at 0.25, 0.5, 1.0 and 2.0 ppm. Also, 0.75% glucose was added to the culture broth at the time of the IPTG addition. Further, 0.3% glucose per hour was additionally fed to the culture broth starting from 30 min after the IPTG addition. The cells were cultured up to 24 hrs after the start of the cultivation. After the IPTG addition, a 0.5 ml sample of the culture broth was taken at regular intervals and centrifuged, followed by storing the resultant cells at -20°C.

To the thus stored frozen cells, 0.5 ml of 0.1 M Tris-hydroxyaminomethane buffer (pH 8.0) containing 7 M guanidine hydrochloride, 10 mM dithiothreitol and 0.1% (w/v) Tween 20 was added and stirred sufficiently. The resultant mixture was sonicated at room temperature for 3 min to thereby extract the cell contents, and then centrifuged at 15000 rpm for 5 min to thereby obtain an extract supernatant. This supernatant was diluted 10-fold with 0.1% (v/v) trifluoroacetic acid solution and centrifuged at 15000 rpm for 10 min. Subsequently, 0.1 ml of the resultant supernatant was subjected to column chromatography using Asahipak C4P-50 4E (Showa Denko K.K.) to thereby quantitatively determine [Cys³⁵]-human PTH (1-84) and its Met addition form. The column chromatography was performed by elution using the density gradients of 0.1% trifluoroacetic acid solution containing solvent A: 0.1% trifluoroacetic acid and solvent B: 80% acetonitrile.

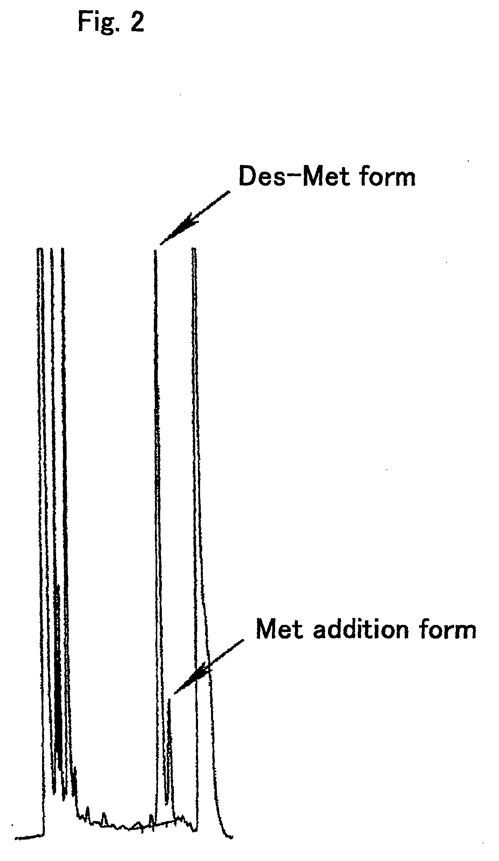

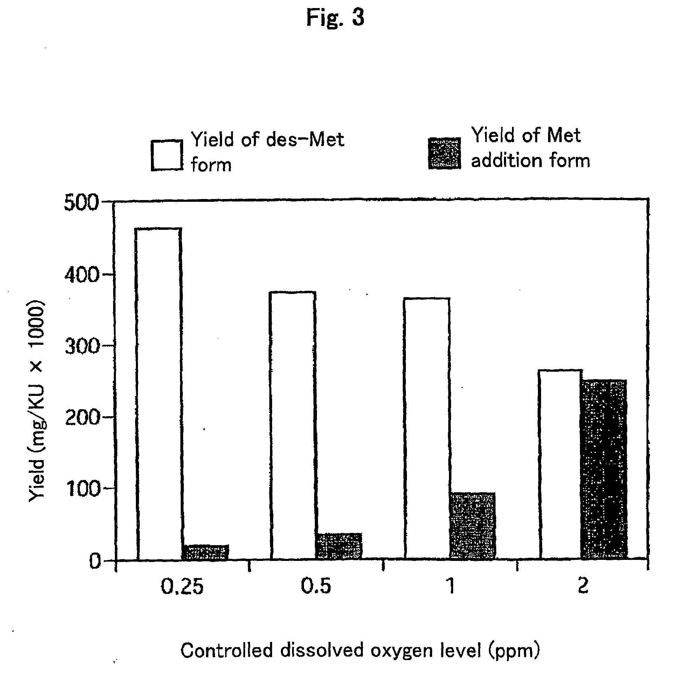

Fig. 2 shows a chart of reversed phase HPLC. Fig. 3 shows the results of quantitative determination of [Cys³⁵]-human PTH (1-84) and its Met addition form in cell extract 14 hrs after the start of the cultivation. The lower the controlled dissolved oxygen value was, the lower the Met addition ratio was and thus a high [Cys³⁵]-human PTH (1-84) productivity was obtained.

### EXAMPLE 2

### Cultivation of [Cys³⁵]-Human PTH (1-84)-Producing Recombinant E. coli in 500-L Fermenters

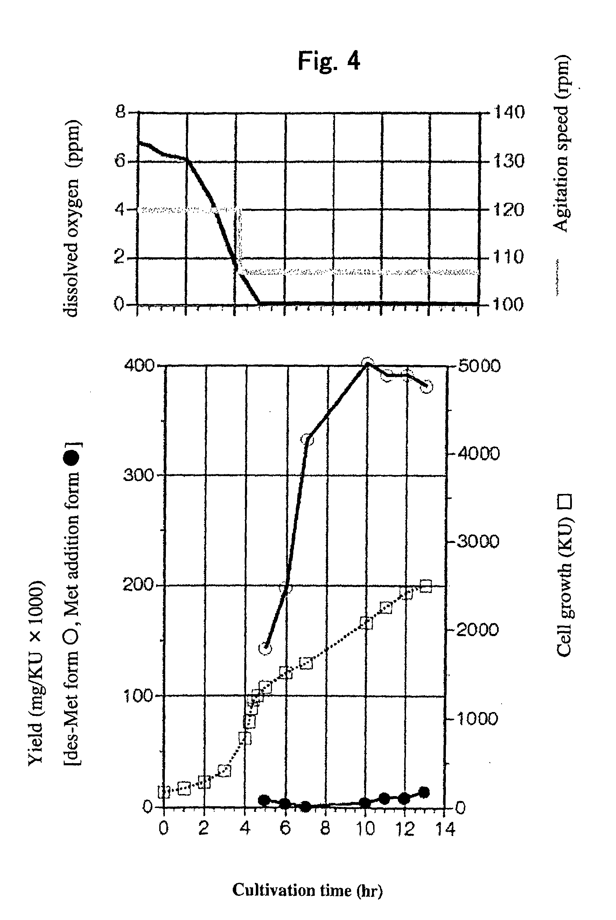

*E. coli* MM294 (DE3)/pTCPTH2 was cultured in 1 L of LB medium (1% peptone, 0.5% yeast extract, 0.5% sodium chloride) containing 10 mg/ml tetracycline at 30°C for 16 hrs under shaking. The resultant culture broth was transferred into a 50-L fermenter containing 20 L of LB medium containing 0.02% New Pole LB-625 and 50 mg/ml ampicillin and cultured at 37°C for 8 hrs under aeration and agitation. The resultant culture broth was transferred into 500-L fermenters each containing 360 L of a medium for primary fermentation (1.68% sodium monohydrogenphosphate, 0.3% sodium dihydrogenphosphate, 0.1% ammonium chloride, 0.05% sodium chloride, 0.024% magnesium sulfate, 0.02% New Pole LB-625, 0.0005% thiamine hydrochloride, 1.5% glucose, and 1.5% casamino acids). Then, aeration agitation culture was started at 37°C with at an aeration rate of 260 L/min and at an agitation speed of 120 rpm. When the turbidity of the culture broth reached approx. 1200 Klett units (KU), isopropyl-β-D-thiogalactopyranoside (IPTG) was added at 5.95 mg/L min. After the IPTG addition, the agitation speed in individual fermenters was changed to 120 and 105 rpm. At the time of the IPTG addition, 0.75% glucose was added to the culture broth. Then, 0.3% glucose per hour was fed to the culture broth from 30 min after the IPTG addition. The cells were cultured up to 24 hrs after the start of the cultivation. After the IPTG addition, a 0.5 ml sample was taken from the culture broth at regular intervals and centrifuged, followed by storing the resultant cells at -20°C. Using the thus stored frozen cells, yield and Met addition ratio were determined in the same manner as in Example 1 (Fig. 4).

The cultivation process of the invention also demonstrated a low Met-addition ratio in the cultivation using 500-L fermenters.

### EXAMPLE 3

### Preparation of PTH (1-34) from [Cys³⁵]-Human PTH (1-84)

[Cys³⁵]-human PTH (1-84) was isolated from cells cultured according to the cultivation process of the invention. After cyanation of the cysteine residue at position 35, the resultant protein was cleaved to thereby obtain human PTH (1-34).

After addition of IPTG, the agitation speed was set at 105 rpm, and cells were cultured in the same manner as described in Example 2. The contents of 3 kg of the resultant cells were extracted with 10 L of 0.1 M Tris-HCl (pH 8.0) supplemented with 6 M guanidine hydrochloride and 0.1 mM APMSF. The extract was centrifuged at 8000 rpm for 20 min, and the resultant supernatant was diluted about 40-fold with 20 mM ammonium acetate buffer (pH 5) containing 1 mM DTT. The resultant dilution was subjected to series of centrifugations at 10000 rpm to remove insoluble matters (precipitate). The resultant supernatant was applied to SP-Toyopearl 650M column (30 cm x 63 cm; Tosoh Corp.) pre-equilibrated with 20 mM ammonium acetate buffer (pH 5) and eluted with linear density gradients of from 0 to 1.0 M NaCl. The resultant eluate was centrifuged and desalted using Pellicon Cassette system (molecular weight cutoff: 1000; Millipore) while adding 0.1 M acetic acid. Urea was added to 2.5 L of the concentrated/desalted solution to give a concentration of 6 M. Then, 2.2 g of 1-cyano-4-dimethylaminopyridinium salt (DMAP-CN) was added thereto and reacted at room temperature for 15 min to thereby carry out S-cyanation. This reaction solution was applied to SP-Toyopearl 650M column (30 cm x 63 cm; Tosoh Corp.) pre-equilibrated with 20 mM ammonium acetate buffer (pH 5) and eluted with linear density gradients of from 0.1 to 1.0 M NaCl. The resultant eluate was centrifuged and desalted using Pellicon Cassette system (molecular weight cutoff: 1000; Millipore). Urea was added to 2 L of the concentrated/desalted solution to give a concentration of 6 M, and then 1 N NaOH was added thereto to give a final concentration of 0.05 N. The resultant solution was ice-cooled for 10 min to thereby carry out a cleavage reaction to cut the protein at the position of the S-cyanated cysteine. Then, the reaction was terminated by adding acetic acid to make the pH 5 or below. The resultant solution was subjected to gel filtration chromatography using Toyopearl HW-50F column (30 cm x 63 cm; Tosoh Corp.) pre-equilibrated with 20 mM ammonium acetate buffer (pH 5). The resultant eluate was adsorbed on the SP-Toyopearl 650S column (20 cm x 32 cm; Tosoh Corp.) pre-equilibrated with 2 M urea-containing 20 mM ammonium acetate buffer (pH 5), and eluted using linear density gradients of 2 M urea-containing 20 mM ammonium acetate buffer (pH 5) and 2 M urea and 0.5 M sodium chloride-containing 20 mM MES buffer (pH 8). The eluate was applied to Phenyl-5PW column (10.8 cm x 20 cm; Tosoh Corp.) pre-equilibrated with 35% saturated ammonium sulfate-containing 20 mM ammonium acetate (pH 5.0) and eluted using linear density gradients of equilibration buffer and 20 mM ammonium acetate (pH 5.0). The eluate was applied to ODS-120T column (30 cm x 75 cm; Tosoh Corp.) pre-equilibrated with 0.1% trifluoroacetic acid and eluted using linear density gradients of 0.1% trifluoroacetic acid solution containing 80% acetonitrile. The eluate was diluted 10-fold with 20 mM ammonium acetate buffer (pH 5), adsorbed on CM-Toyopearl 650 M column (4 cm x 50 cm; Tosoh Corp.) pre-equilibrated with 20 mM ammonium acetate buffer (pH 5), and eluted using linear density gradients of 20 mM ammonium acetate buffer (pH 5) and 1 M sodium chloride-containing 20 mM Tris-HCl buffer (pH 8). The eluate was applied to Sephadex G-10 column (14 cm x 65 cm; Amersham Pharmacia Biotech) pre-equilibrated with distilled water and then eluted with distilled water to thereby obtain 5.88 g of PTH (1-34).

PTH (1-34) was subjected to vapor-phase hydrolysis using 6 N hydrochloric acid solution containing 4% thioglycolic acid at 110°C for 24 and 48 hrs. Then, the amino acid composition was determined with an amino acid analyzer (Hitachi L-8500A Amino Acid Analyzer) (Table 1; in this Table, the values for Ser and Thr are extrapolated to the values at time 0 hr. The values for other amino acids are the mean values of those at time 24 hr and 48 hr.)

The N-terminal amino acid sequence of PTH (1-34) was determined with a vapor-phase protein sequencer (Applied Biosystems Model 477A). As a result, the determined sequence was consistent with the amino acid sequence predicted from the cDNA sequence; addition of Met to the N-terminal was not observed (Table 2).

**Table 1**

| Amino acid | Measured Value | Theoretical Value |
|---|---|---|
| Asx | 3.9 | 4 |
| Ser | 2.8 | 3 |
| Glx | 4.9 | 5 |
| Gly | 1.0 | 1 |
| Val | 3.2 | 3 |
| Met | 1.8 | 2 |
| Ile | 1.0 | 1 |
| Leu | 5.0 | 5 |
| Phe | 1.0 | 1 |
| Lys | 3.0 | 3 |
| His | 2.8 | 3 |
| Arg | 2.0 | 2 |
| Trp | 0.9 | 1 |

**Table 2**

| No. | Detected PTH Amino Acid (p mole) | Amino Acid Predicted from Nucleotide Sequence |
|---|---|---|
| 1 | Ser (249) | Ser |
| 2 | Val (576) | Val |
| 3 | Ser (262) | Ser |
| 4 | Glu (308) | Glu |
| 5 | Ile (542) | Ile |
| 6 | Gln (412) | Gln |
| 7 | Leu (392) | Leu |
| 8 | Met (407) | Met |
| 9 | His (79) | His. |
| 10 | Asn (314) | Asn |
| 11 | Leu (321) | Leu |
| 12 | Gly (243) | Gly |
| 13 | Lys (262) | Lys |
| 14 | His (94) | His |
| 15 | Leu (240) | Leu |
| 16 | Asn (59) | Asn |
| 17 | Ser (44) | Ser |
| 18 | Met (162) | Met |
| 19 | Glu (50) | Glu |
| 20 | Arg (78) | Arg |

### REFERENCE EXAMPLE 2

### Construction of Plasmids for Searching for Optimized Sequences

### 1) Construction of Plasmid pGFPuvqc

In order to eliminate the NdeI restriction site located in the GFPuv structural gene in pGFPuv (Clontech), the codon of the His at position 77 was changed from CAT to CAC through site-directed mutagenesis (QuickChange™ Site Directed Mutagenesis Kit, Stratagene) using primer 1: CGTTATCCGGATCACATGAAACGGCATG (SEQ ID NO: 1) and primer 2: CATGCCGTTTCATGTGATCCGGATAACG (SEQ ID NO: 2). Thus, plasmid pGFPuvqc was obtained.

### 2) Construction of Plasmid pGFPuvqd

Plasmid pGFPuvqc was digested with HindIII (Takara Shuzo) and KpnI (Takara Shuzo), followed by agarose gel electrophoresis to cut out an approx. 3.3 kbp band. The DNA was recovered from the gel using QIAquick Gel Extraction Kit (Qiagen). Synthetic DNA 1: AGCTTCATATGTTCGAAGTACTAGATCTGGTAC (SEQ ID NO: 3) and synthetic DNA 2: CAGATCTAGTACTTCGAACATATGA (SEQ ID NO: 4) (100 pmol each) were dissolved in TE buffer, retained at 90°C for 10 min, and then gradually cooled to room temperature to perform annealing. The thus annealed DNA fragment was inserted into pGFPuvqc between the HindIII and KpnI sites using Ligation kit ver. 2 (Takara Shuzo) to thereby obtain plasmid pGFPuvqd.

### 3) Construction of Plasmid pGFPNP

Plasmid pNPHGH in which the T7 promoter of pTCHGH-Na described in Reference Example 1 in WO 00/20439 is replaced with a synthetic promoter NP2 was constructed as described below. Briefly, plasmid pTCHGH-Na was digested with EcoRI (Takara Shuzo) and XbaI (Takara Shuzo), followed by agarose gel electrophoresis to cut out an approx. 4.6 kbp band. The DNA was recovered from the gel using QIAquick Gel Extraction Kit (Qiagen). One hundred pmol each of synthetic DNA 3: AATTCTATAAAAATAATTGTTGACATATTTTATAAATTTTGGCATAATAGATCTAAT TGTGAGCGGATAACAATTCTGCAGAAGCTTGAGCTCGGTACCCGGGGATCCT (SEQ ID NO: 5) comprising the synthetic promoter NP2, and synthetic DNA 4: CTAGAGGATCCCCGGGTACCGAGCTCAAGCTTCTGCAGAATTGTTATCCGCTCA CAATTAGATCTATTATGCCAAAATTTATAAAATATGTCAACAATTATTTTTATAG (SEQ ID NO: 5) comprising a nucleotide sequence complementary to the synthetic promoter NP2 were dissolved in TB buffer, retained at 90°C for 10 min, and then gradually cooled to room temperature to perform annealing. The resultant double-stranded DNA fragment was inserted into pTCHGH-Na between the EcoRI and XbaI sites using Ligation kit ver. 2 (Takara Shuzo) to thereby obtain plasmid pNPHGH.

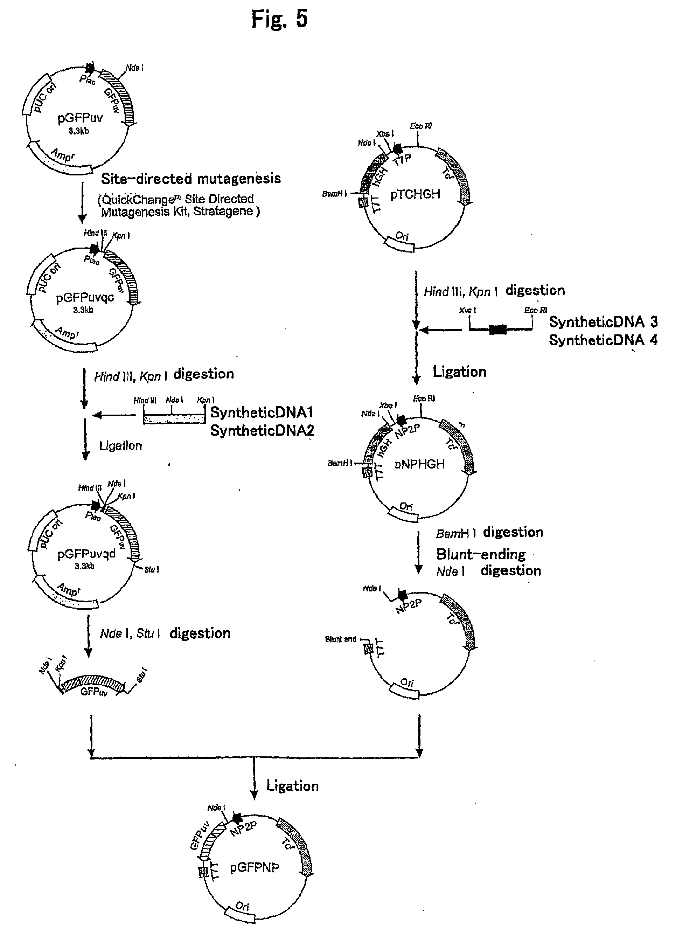

Plasmid pNPHGH was digested with BamHI (Takara Shuzo), followed by blunt-ending with DNA Blunting Kit (Takara Shuzo). After further digestion with NdeI, the digest was subjected to agarose gel electrophoresis to cut out an approx. 4.6 kbp band. The DNA was recovered from the gel using QIAquick Gel Extraction Kit (Qiagen). Plasmid pGFPuvqd was digested with NdeI and StuI, followed by agarose gel electrophoresis to cut out an approx. 0.8 kbp band. A DNA comprising the GFPuv structural gene was recovered from the gel using QIAquick Gel Extraction Kit (Qiagen). The thus recovered DNA was inserted into pNPHGH between the NdeI site and BamHI (blunted) site using Ligation kit ver. 2 (Takara Shuzo) to thereby obtain plasmid pGFPNP for use in selecting N-terminal optimized sequences (Fig. 5).

Plasmid pGFPNP is an expression plasmid containing a GFPuv structural gene to be transcribed from NP2 promoter and having NdeI, Scal and AgeI restriction sites upstream of the GFPuv structural gene. It is possible to insert DNA fragments into this plasmid using these restriction enzyme sites.

*E. coli* JM109 was transformed with plasmid pGFPNP to thereby obtain *E. coli* JM109/pGFPNP.

### REFERENCE EXAMPLE 3

### Search for Rat GALP Sequences Optimized for Expression

Plasmid pGFPNP was digested with NdeI (Takara Shuzo) and Scal (Takara Shuzo), followed by agarose gel electrophoresis to cut out an approx. 4.9 kbp band. The DNA was recovered from the gel using QIAquick Gel Extraction Kit (Qiagen).

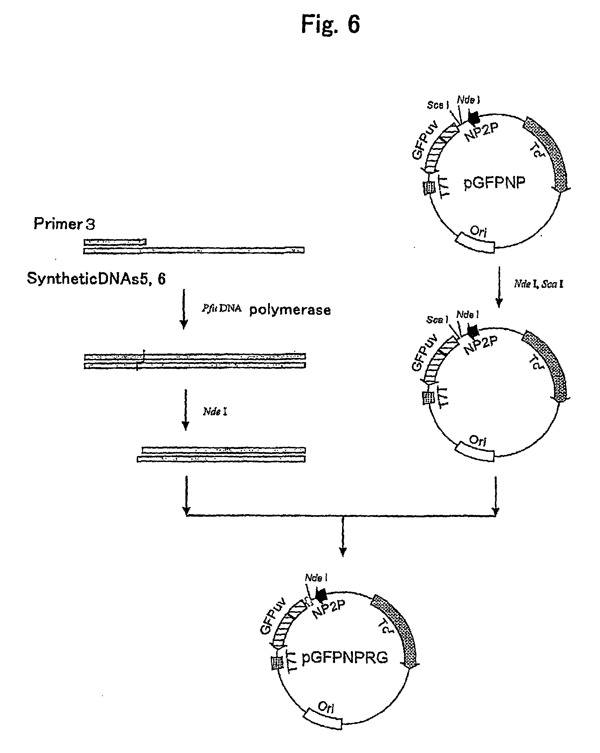

Random nucleotide sequences encoding N-terminal amino acids of rat GALP (a rat type ligand peptide for rat galanin receptor (1-60); WO 99/48920) were prepared as described below. Briefly, double-stranded DNA fragments were prepared with *Pfu* DNA polymerase (Stratagene) using mixed bases-containing synthetic DNA 5: AGGTAACCAGCACTATTTAAAGTCCANCCNCCNCGNCCNCGRTGNGCNGGNGC CATATGTATATCTCCTTCTTAAAG (SEQ ID NO: 7) and synthetic DNA 6: AGGTAACCAGCACTATTTAAAGTCCANCCNCCYCTNCCYCTRTGNGCNGGNGC CATATGTATATCTCCTTCTTAAAG (SEQ ID NO: 8) as templates and primer 3: CTTTAAGAAGGAGATATACATATG (SEQ ID NO: 9) as a primer. The extension reaction solution was composed of 5 µl of *Pfu* DNA polymerase, 5 µl each of 10x Reaction buffer attached to the polymerase and dNTP mixture (Takara Shuzo), 1 µl each of synthetic DNA 3 (42.2 µmol/L), synthetic DNA 4 (5 µmol/L) and primer 3 (100 µmol/L), and 32 µl of distilled water. The extension reaction was performed at 94°C for 30 sec, at 58°C for 30 sec, and at 72°C for 20 min. The resultant reaction solution was concentrated and desalted with Microcon 30 (Nihon Millipore), followed by digestion of resultant double-stranded DNA fragments with NdeI (Takara Shuzo). This digestion with NdeI was conducted as described below. Briefly, a reaction solution consisting of 10 µl of the above concentrated/desalted reaction solution, 2 µl of Buffer H (Takara Shuzo), 7 µl of distilled water and 1 µl of NdeI was retained at 37°C for 3 hr to perform digestion. From the reaction solution after this NdeI digestion, DNA fragments were purified using QIAquick Gel Extraction Kit (Qiagen). These DNA fragments were inserted individually into pGFPNP2 between the NdeI and Scal sites using Ligation kit ver. 2 (Takara Shuzo) to thereby prepare a set of expression plasmid pGFPNPRG having at the N-terminal of GFPuv a random nucleotide sequence encoding N-terminal amino acids of rat GALP (Fig. 6).

*E. coli* JM109 was transformed with this set of expression plasmid pGFPNPRG, coated on LB agar medium containing 10 mg/L tetracycline, and cultured overnight at 37°C for colony formation. The thus formed colonies were observed under a UV ramp at 354 nm, followed by selection of 2 colonies emitting intense fluorescence and 16 colonies emitting weak fluorescence. These colonies were subjected to colony PCR using primer 4: TTCATACACGGTGCCTGACTGCGTTAG (SEQ ID NO: 10) and primer 5: TCAACAAGAATTGGGACAACTCC (SEQ ID NO: 11), followed by agarose gel electrophoresis to confirm that a DNA fragment of interest is inserted. The products of this colony PCR were purified with QIAquick PCR Purification Kit (Qiagen), and their nucleotide sequences were analyzed with primers 4 and 5. As a result, the nucleotide sequences shown in Table 3 were obtained. Of these, the nucleotide sequences No. 1 and No. 2 were obtained from those *E. coli* clones emitting intense fluorescence.

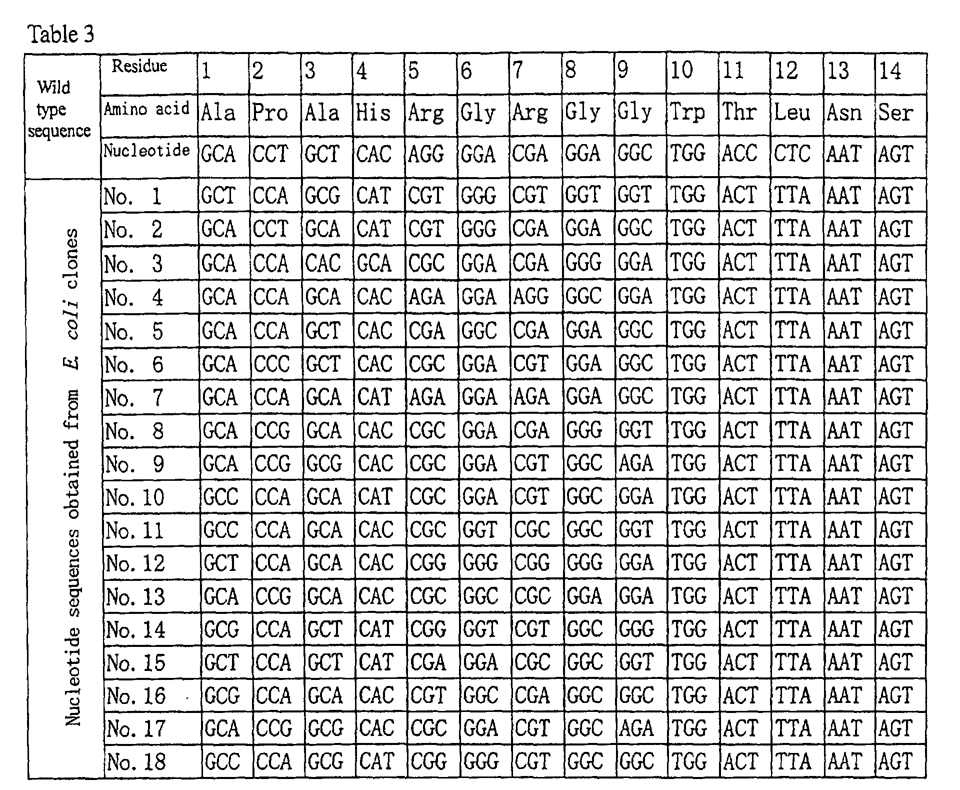

(N-terminal Met was taken as residue number 0.)

### REFERENCE EXAMPLE 4

### Construction of Clones that Express Rat GALP Encoded by Wild Type Nucleotide Sequence

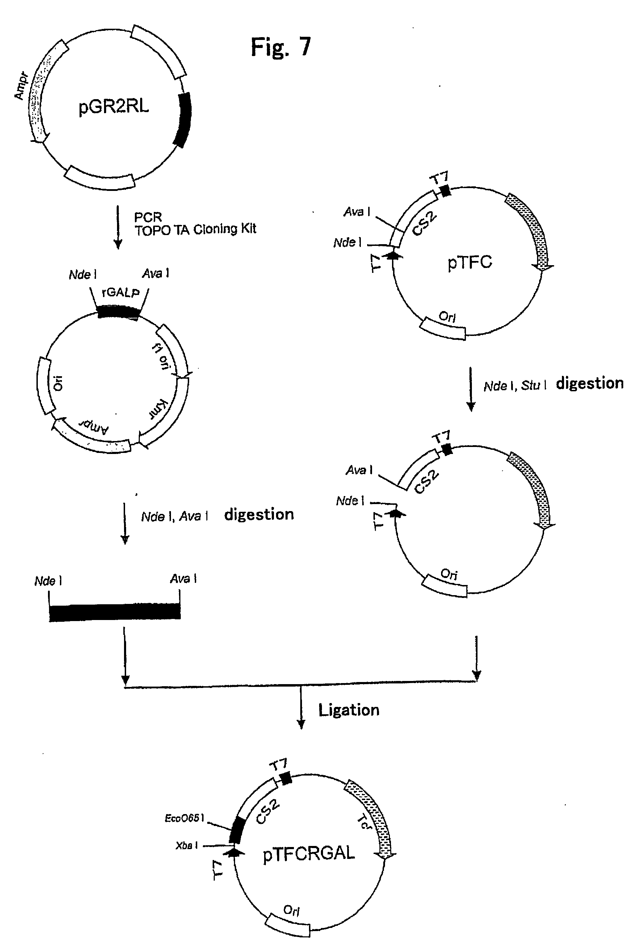

As a rat GALP expression plasmid, plasmid pTFCRGAL was constructed as described below for expression of rat GALP-CS23 fusion protein (fusion protein of rat GALP and human bFGF mutein CS23 (EP0281822)) from T7 promoter (Fig. 7).

Plasmid pTFC described in Example 21 in WO 99/48920 was digested with NdeI and AvaI, followed by agarose gel electrophoresis to cut out an approx. 3.9 kbp band. The DNA was recovered from the gel using QIAquick Gel Extraction Kit (Qiagen).

A fragment containing a rat GALP structural gene was amplified by PCR from plasmid pGR2RL4 (described in Example 18 in WO 99/48920) where a rat GALP structural gene is cloned, using primer 6: AGCATATGGCACCTGCTCACAGG (SEQ ID NO: 12) having an initiation codon ATG and an NdeI restriction site upstream of rat GALP structural gene and primer 7: CCCTCGGGGCAGGTCATCCTTGGAGAG (SEQ ID NO: 13) having a TGC codon encoding Cys and an Aval restriction site downstream of the GALP structural gene. The resultant fragment was cloned using TOPO TA Cloning Kit to thereby obtain plasmid pCR2.1TOPO/rGAL. This plasmid pCR2.1TOPO/rGAL was digested with NdeI and Aval, followed by agarose gel electrophoresis to cut out an approx, 0.2 kbp band. The DNA was recovered from the gel using QIAquick Gel Extraction Kit (Qiagen). This DNA fragment was ligated to pTFC between the NdeI and AvaI sites using Ligation kit ver. 2 (Takara Shuzo) to thereby obtain plasmid pTFCRGAL.

*E. coli* MM294 (DE3) was transformed with this expression plasmid pTFCRGAL to thereby obtain *E. coli* MM294 (DE3)/pTFCRGAL having the ability to express rat GALP-CS23 fusion protein.

### REFERENCE EXAMPLE 5

### Expression of Rat GALP using Optimized Sequences

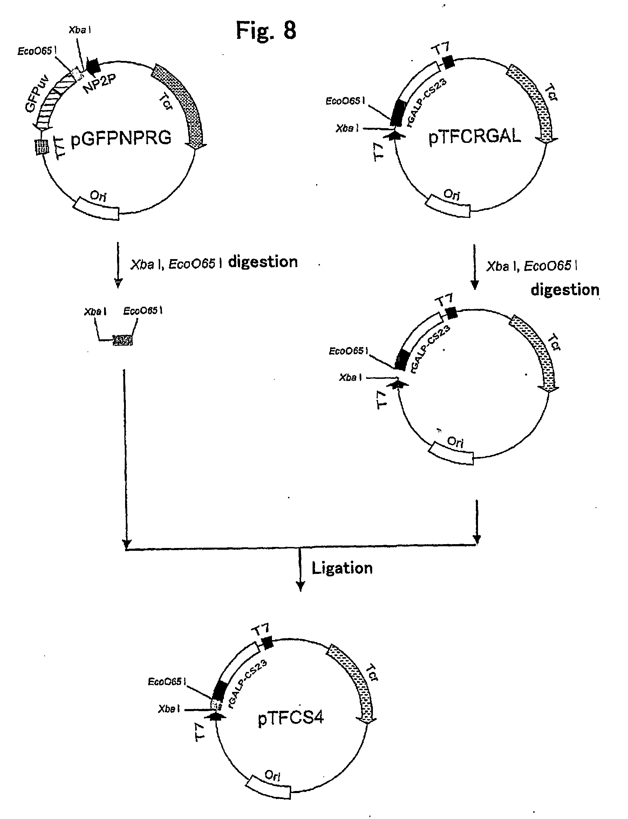

A nucleotide sequence encoding an N-terminal portion of the rat GALP structural gene in pTFCRGAL was changed from the corresponding wild type sequence to the nucleotide sequence obtained from an intense fluorescence-emitting colony in Referene Example 3, as described below (Fig. 8).

Rat GALP-CS23 expression plasmid pTFCRGAL encoding the wild-type nucleotide sequence was digested with Xbal and EcoO65I, followed by agarose gel electrophoresis to cut out an approx. 4.5 kbp band. The DNA was recovered from the gel using QIAquick Gel Extraction Kit (Qiagen). Colony No. 1 obtained in Reference Example 3 was inoculated into LB medium containing 10 mg/L tetracycline and cultured overnight at 37°C. From the resultant cells, plasmid pGFPNP/rGAL1 was purified using QIAprep8 Miniprep Kit (Qiagen). This plasmid pGFPNP/rGAL1 was digested with XbaI and Eco065I, followed by agarose gel electrophoresis to cut out an approx. 0.1 kbp band. The DNA was recovered from the gel using QIAquick Gel Extraction Kit (Qiagen). This DNA fragment was ligated to pTFCRGAL between the XbaI and EcoO65I sites using Ligation kit ver. 2 (Takara Shuzo) to thereby obtain plasmid pTFCS4. This is an expression plasmid for rat GALP-CS23 fusion protein having the nucleotide sequence obtained from clone No. 1 selected in Reference Example 3 at an N-terminal portion of rat GALP.

*E. coli* MM294 (DE3) was transformed with this expression plasmid pTFCS4 to thereby obtain *E. coli* MM294 (DE3)/pTFCS4 having the ability to express rat GALP-CS23 fusion protein.

### EXAMPLE 4

### Cultivation of Rat GALP-CS23 Fusion Protein-Producing Recombinant E. coli in 3-L Jars

The above-described *E. coli* MM294 (DE3)/pTFCS4 was cultured in 1 L of LB medium (1% peptone, 0.5% yeast extract, 0.5% sodium chloride) containing 10 mg/ml tetracycline at 30°C for 16 hrs under shaking. The resultant culture broth was transferred into 3-L jars each containing 1.5 L of a medium for primary fermentation (1.68% sodium monohydrogenphosphate, 0.3% sodium dihydrogenphosphate, 0.1% ammonium chloride, 0.05% sodium chloride, 0.024% magnesium sulfate, 0.02% New Pole LB-625, 0.0005% thiamine hydrochloride, 1.5% glucose, and 1.5% casamino acids). Then, aeration agitation culture was started at 37°C at an aeration rate of 1.5 L/min and at an agitation speed of 500 rpm. When the dissolved oxygen concentration became 2.0 ppm or below, the agitation speed was controlled so that the dissolved oxygen concentration is retained at 2.0 ppm. When the turbidity of the culture broth reached approx. 2400 Klett units, isopropyl-β-D-thiogalactopyranoside (IPTG) was added at 5.95 mg/L min. From immediately after the addition of IPTG, cultivation was continued in one jar where the dissolved oxygen concentration was controlled at 2.0 ppm, while cultivation was continued in another jar where the agitation speed was fixed at 550 rpm to retain the dissolved oxygen concentration in the culture broth at a low level. Also, 0.75% glucose was added to the culture broth at the time of the IPTG addition. Further, 0.3% glucose per hour was additionally fed to the culture broth starting from 30 min after the IPTG addition. The cells were cultured up to 24 hrs after the start of the cultivation. After the IPTG addition, a 0.5 ml sample of the culture broth was taken at regular intervals and centrifuged, followed by storing the resultant cells at -20°C.

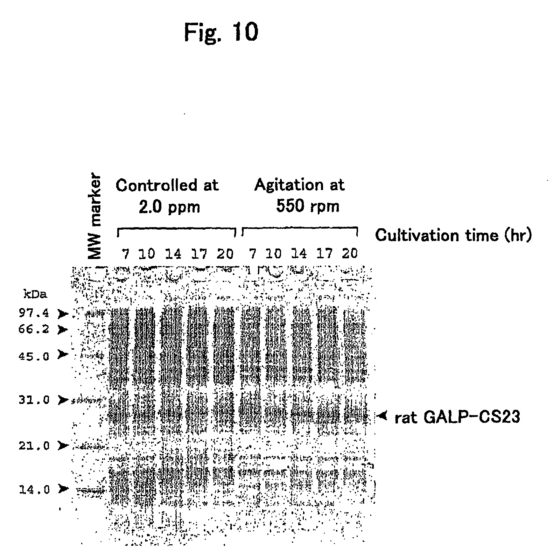

The thus stored frozen cells were thawed and suspended in 0.5 ml of 20 mmol/L Tris-HCl buffer (pH 7) containing 0.15 mol/L sodium chloride. Fifty µl of the suspension was mixed with a sample buffer (125 mM Tris-HCl, 1% sodium dodecyl sulfate, 15% glycerol, 5% 2-mercaptoethanol, 0.005% Bromophenol Blue) and electrophoresed on Multi-gel 15/25 (Daiichi Pure Chemicals). The resultant gel was stained with Rapid CBB KANTO (Kanto Kagaku). The results are shown in Fig. 10. No significant difference was observed in the expression level between the two cultivation conditions.

### EXAMPLE 5

### Analysis of the N-Terminal Amino Acids of the Rat GALP-CS23 Fusion Protein Expressed in Example 4

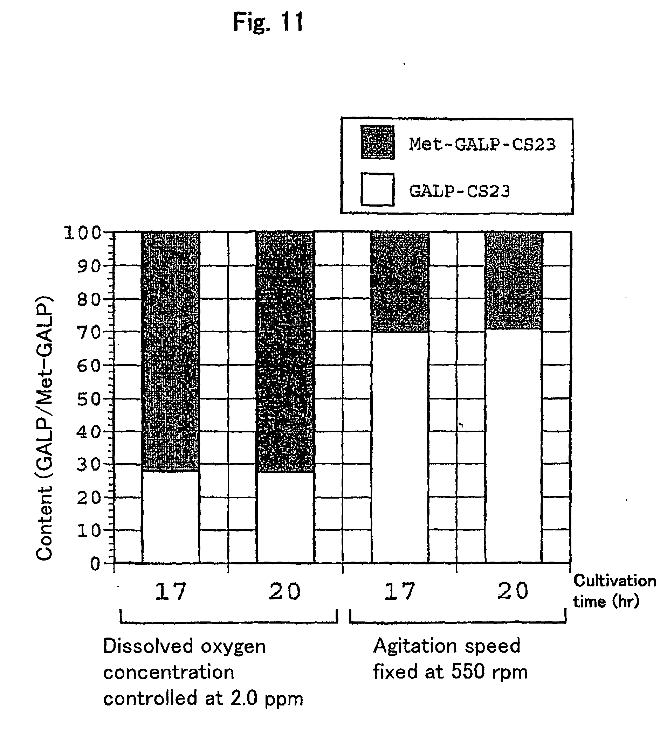

The cells stored frozen in Example 4 were thawed, suspended in 0.5 ml of 20 mmol/L Tris-HCl buffer (pH 7) containing 0.15 mol/L sodium chloride, disrupted with a sonicator (Olympus) and centrifuged at 15000 rpm for 5 min to thereby obtain rat GALP-CS23 fusion protein expressed as an inclusion body. This inclusion body was dissolved in a sample buffer (125 mM Tris-HCl, 1% sodium dodecyl sulfate, 15% glycerol, 5% 2-mercaptoethanol, 0.005% Bromophenol Blue) and then electrophoresed on Multi-gel 15/25 (Daiichi Pure Chemicals). The protein was transferred from the gel onto a PVDF membrane. A band corresponding to rat GALP-CS23 fusion protein was cut out. The N-terminal amino acids of this protein were determined with a vapor-phase protein sequencer (Applied Biosystems Model 472). Then, the ratios of Met form and Des-Met form were calculated. Fig. 11 shows the results of measurement at 17 and 20 hrs after the start of the cultivation. When the dissolved oxygen concentration was controlled at 2.0 ppm, addition of Met was observed in about 70% of the protein. On the other hand, when the agitation speed after the IPTG addition was fixed at 550 rpm, the percentage of Met-added protein decreased to about 30%; thus, the effect of removal of N-terminal Met according to the cultivation process of the invention was observed.

### INDUSTRIAL APPLICABILITY

According to the present invention, expression of a recombinant protein from which the N-terminal Met has been removed is possible at a high efficiency.

## Claims

1. A process of producing a recombinant protein in which methionine is not added to the N-terminal comprising culturing a host transformed with an expression vector for expressing the recombinant protein, wherein the process is **characterized by** controlling the dissolved oxygen concentration in the culture broth at about 1 ppm or below.

2. The process according to claim 1, wherein the dissolved oxygen concentration in the culture broth is about 0.01-1.0 ppm.

3. The process according to claim 1, wherein the dissolved oxygen concentration in the culture broth is 0.05-1.0 ppm.

4. The process according to claim 1, wherein the dissolved oxygen concentration in the culture broth is controlled at about 1 ppm or below by regulating the agitation speed in the culture broth.

5. The process according to claim 1, wherein the host is a bacterium belonging to the genus *Escherichia.*

6. The process according to claim 1, wherein an expression inducer is added to the culture broth.

7. The process according to claim 6, wherein the expression inducer is isopropyl-β-D-thiogalactopyranoside.

8. The process according to claim 6, wherein the concentration of the expression inducer in the culture broth is about 0.01-1 mM.


SEQUENCE LISTING

<110> Takeda Chemical Industries, Ltd.

<120> Method of Culture for Recombinant Escherichia coli.

<130> P2001-183PCT

<150> JP 2000-232389
<151> 2000-7-31

<160> 18

<210> 1
<211> 28
<212> DNA
<213> Artificial sequence

<220>
<223> Synthetic DNA

<400> 1
cgttatccgg atcacatgaa acggcatg    28

<210> 2
<211> 28
<212> DNA
<213> Artificial sequence

<220>
<223> Synthetic DNA

<400> 2
catgccgttt catgtgatcc ggataacg    28

<210> 3

<211> 33
<212> DNA
<213> Artificial sequence

<220>
<223> Synthetic DNA

<400> 3
agcttcatat gttcgaagta ctagatctgg tac    33

<210> 4
<211> 25
<212> DNA
<213> Artificial sequence

<220>
<223> Synthetic DNA

<400> 4
cagatctagt acttcgaaca tatga    25

<210> 5
<211> 109
<212> DNA
<213> Artificial sequence

<220>
<223> Synthetic DNA

<400> 5
aattctataa aaataattgt tgacatattt tataaatttt ggcataatag atctaattgt  60
gagcggataa caattctgca gaagcttgag ctcggtaccc ggggatcct              109

<210> 6
<211> 109
<212> DNA

<213> Artificial sequence

<220>
<223> Synthetic DNA

<400> 6
ctagaggatc cccgggtacc gagctcaagc ttctgcagaa ttgttatccg ctcacaatta 60
gatctattat gccaaaattt ataaaatatg tcaacaatta tttttatag 109

<210> 7
<211> 77
<212> DNA
<213> Artificial sequence

<220>
<223> Synthetic DNA

<400> 7
aggtaaccag cactatttaa agtccanccn ccncgnccnc grtgngcngg ngccatatgt 60
atatctcctt cttaaag 77

<210> 8
<211> 77
<212> DNA
<213> Artificial sequence

<220>
<223> Synthetic DNA

<400> 8
aggtaaccag cactatttaa agtccanccn ccyctnccyc trtgngcngg ngccatatgt 60
atatctcctt cttaaag 77

<210> 9
<211> 24
<212> DNA

<213> Artificial sequence

<220>
<223> Synthetic DNA

<400> 9
ctttaagaag gagatataca tatg   24

<210> 10
<211> 27
<212> DNA
<213> Artificial sequence

<220>
<223> Synthetic DNA

<400> 10
ttcatacacg gtgcctgact gcgttag   27

<210> 11
<211> 23
<212> DNA
<213> Artificial sequence

<220>
<223> Synthetic DNA

<400> 11
tcaacaagaa ttgggacaac tcc   23

<210> 12
<211> 23
<212> DNA
<213> Artificial sequence

<220>

<223> Synthetic DNA

<400> 12
agcatatggc acctgctcac agg    23

<210> 13
<211> 27
<212> DNA
<213> Artificial sequence

<220>
<223> Synthetic DNA

<400> 13
ccctcggggc aggtcatcct tggagag    27

<210> 14
<211> 84
<212> PRT
<213> Human

<400> 14
Ser Val Ser Glu Ile Gln Leu Met His Asn Leu Gly Lys His Leu Asn
1               5                   10                  15
Ser Met Glu Arg Val Glu Trp Leu Arg Lys Lys Leu Gln Asp Val His
            20                  25                  30
Asn Phe Cys Ala Leu Gly Ala Pro Leu Ala Pro Arg Asp Pro Gly Ser
        35                  40                  45
Gln Arg Pro Arg Lys Lys Glu Asp Asn Val Leu Val Glu Ser His Glu
    50                  55                  60
Lys Ser Leu Gly Glu Ala Asp Lys Ala Asp Val Asn Val Leu Thr Lys
65                  70                  75                  80
Ala Lys Ser Gln
            84

<210> 15

<211> 258
<212> DNA
<213> Human

<400> 15
atgtctgtgt ccgagattca gttaatgcat aaccttggca aacatttgaa ctcgatggag 60
cgtgtagaat ggctgcgtaa gaagttgcag gatgtgcaca atttttgcgc cttaggtgcc 120
ccattggctc ctcgtgatcc tggttcccaa agaccacgta aaaaggaaga caatgtctta 180
gttgagagcc atgaaaaatc cctaggcgag gcagacaagg ccgatgtgaa tgtattaact 240
aaagctaaat cccagtaa 258

<210> 16
<211> 34
<212> PRT
<213> Human

<400> 16
Ser Val Ser Glu Ile Gln Leu Met His Asn Leu Gly Lys His Leu Asn
1               5                   10                  15
Ser Met Glu Arg Val Glu Trp Leu Arg Lys Lys Leu Gln Asp Val His
            20                  25                  30
Asn Phe
34

<210> 17
<211> 204
<212> PRT
<213> Rat

<400> 17
Ala Pro Ala His Arg Gly Arg Gly Gly Trp Thr Leu Asn Ser Ala Gly
1               5                   10                  15
Tyr Leu Leu Gly Pro Val Leu His Leu Ser Ser Lys Ala Asn Gln Gly
            20                  25                  30
Arg Lys Thr Asp Ser Ala Leu Glu Ile Leu Asp Leu Trp Lys Ala Ile
        35                  40                  45

Asp Gly Leu Pro Tyr Ser Arg Ser Pro Arg Met Thr Cys Pro Glu Asp
    50                  55                  60
Gly Gly Ser Gly Ala Phe Pro Pro Gly His Phe Lys Asp Pro Lys Arg
65                  70                  75                  80
Leu Tyr Cys Lys Asn Gly Gly Phe Phe Leu Arg Ile His Pro Asp Gly
                85                  90                  95
Arg Val Asp Gly Val Arg Glu Lys Ser Asp Pro His Ile Lys Leu Gln
            100                 105                 110
Leu Gln Ala Glu Glu Arg Gly Val Val Ser Ile Lys Gly Val Ser Ala
        115                 120                 125
Asn Arg Tyr Leu Ala Met Lys Glu Asp Gly Arg Leu Leu Ala Ser Lys
    130                 135                 140
Ser Val Thr Asp Glu Cys Phe Phe Phe Glu Arg Leu Glu Ser Asn Asn
145                 150                 155                 160
Tyr Asn Thr Tyr Arg Ser Arg Lys Tyr Thr Ser Trp Tyr Val Ala Leu
                165                 170                 175
Lys Arg Thr Gly Gln Tyr Lys Leu Gly Ser Lys Thr Gly Pro Gly Gln
            180                 185                 190
Lys Ala Ile Leu Phe Leu Pro Met Ser Ala Lys Ser
        195                 200             204

<210> 18
<211> 612
<212> DNA
<213> Rat

<400> 18
gcaccagcgc atcgtgggcg tggtggttgg actttaaata gtgctggtta cctcctgggt  60
cctgtcctcc acctttcctc aaaggccaac cagggcagga agacagactc agctcttgag 120
atcctagacc tgtggaaggc catagatggg ctcccttatt cccgctctcc aaggatgacc 180
tgccccgagg atggcggcag cggcgccttc ccgcccggcc acttcaagga ccccaagcgg 240
ctgtactgca aaaacggggg cttcttcctg cgcatccacc ccgacggccg agttgacggg 300
gtccgggaga agagcgaccc tcacatcaag ctacaacttc aagcagaaga gagaggagtt 360
gtgtctatca aaggagtgag cgctaatcgt tacctggcta tgaaggaaga tggaagatta 420
ctagcttcta agtctgttac ggatgagtgt ttcttttttg aacgattgga atctaataac 480
tacaatactt accggtcaag gaaatacacc agttggtatg tggcactgaa acgaactggg 540

cagtataaac ttggatccaa aacaggacct gggcagaaag ctatactttt tcttccaatg 600
tctgctaaga gc 612